**Europäisches Patentamt**

(19) **European Patent Office**

**Office européen des brevets**

(11) **EP 0 696 744 A2**

(12) # EUROPEAN PATENT APPLICATION

(43) Date of publication:
14.02.1996 Bulletin 1996/07

(51) Int. Cl.$^6$: **G01R 35/00**

(21) Application number: **94112037.0**

(22) Date of filing: **05.12.1988**

(84) Designated Contracting States:
**CH DE FR GB LI NL**

(30) Priority: **07.12.1987 US 130046**
**22.08.1988 US 234360**

(71) Applicant: **GENERAL ELECTRIC COMPANY**
**Schenectady, NY 12345 (US)**

(72) Inventors:
• **Roemer, Peter Bernard**
**New York 12309 (US)**
• **Edelstein, William Alan**
**New York 12309 (US)**
• **Hayes, Cecil Edward**
**Wisconsin 53222 (US)**

(74) Representative: **Pratt, Richard Wilson et al**
**London WC2R 3AA (GB)**

(54) **Nuclear magnetic resonance (NMR) imaging with multiple surface coils**

(57)    A method for simultaneously receiving a different NMR response signal from each of a plurality of closely-spaced surface coils, first provides an array of a plurality of the surface coils, each positioned so as to have substantially no interaction with all adjacent surface coils. A different NMR response signal is received from an associated portion of the sample enclosed within an imaging volume defined by the array. Each different NMR response signal is used to construct a different one of a like plurality of NMR images of the sample, with the plurality of different images then being combined, on a point-by-point basis, to produce a single composite NMR image of a total sample portion from which NMR response signal contribution was received by any of the array of surface coils. Interactions between non-adjacent surface coils are minimized by coupling each onto an associated preamplifier.

A nuclear magnetic resonance (NMR) signal acquisition apparatus includes a cylindrical array of overlapping coils. Coupling of currents between coils due to re-radiation of received signals, in particular noise currents, is reduced by presenting a high impedance to each coil, thereby reducing the current circulating in each coil. A PREDAMP circuit is disclosed which utilizes the input impedance of a preamplifier, transformed through a quarter-wavelength transmission line segment, to achieve the high input impedance for the coil. As a result, multiple images, each with a high signal-to-noise ratio (SNR), can be simultaneously obtained. A method is disclosed for combining the multiple images into a composite image with optimum SNR, taking into account the phase shifts between images resulting from the spatial orientation of the coils.

## Description

### Background of the Invention

The present invention relates to nuclear magnetic resonance (NMR) imaging and, more particularly, to methods and apparatus for simultaneously receiving a different NMR response signals from each of a plurality of closely-positioned radio-frequency (RF) coils, having substantially reduced interactions therebetween.

Present-day NMR imaging systems utilize receiver coils which surround the entire sample (for example, a human patient) which is to be imaged (see for example US-A-4707664). These "remote coils" have the advantage that the sensitivity to individual spins is, to a first approximation, substantially constant over the entire region being imaged. Although this uniformity is not strictly characteristic of such remote coils, it is substantially constant to a sufficient degree that most present-day reconstruction techniques assume a constant coil sensitivity. Because of their large size, such remote coils suffer from two disadvantages: first, a relative insensitivity to individual spins: and, second, a relatively large inductance and, therefore, a low self-resonant frequency. It is well known that surface coils do not have a uniform sensitivity to individual spins within the region; images produced using surface coils require additional compensation for such inhomogeneity. Surface coils can, however, be made much smaller in geometry than remote coils and for medical diagnostic use can be applied near, or on, the body surface of the sample patient. This is especially important where attention is being directed to imaging a small region within the same, rather than an entire anatomical cross section. Because the surface coil reception element can be located closer to the spins of interest, a given spin will produce a larger EMF, at a given Larmor frequency, in a surface coil than in a remote coil. The use of a surface coil also reduces the noise contribution from electrical losses in the body, with respect to a corresponding remote coil, while maximizing the desired signal.

NMR imaging Systems thus typically use a small surface coil for localized high resolution imaging. A single surface coil of diameter D gives the highest possible signal-to-noise ratio (SNR) for that volume around a depth D inside an infinite conducting half space. However, the single surface coil can only effectively image that region with lateral dimensions comparable to the surface coil diameter D. Therefore, the use of a surface coil necessarily restricts the field-of-view and inevitably leads to a trade-off between resolution and field-of-view. Since the fundamental limitation of the SNR of a surface coil is its intrinsic signal-to-noise ratio, wherein the noise resistance is attributable to currents induced in the sample (for example, a patient in a medical NMR imaging situation) by the radio-frequency (RF) receiving coil. Larger coils induce greater patient sample losses and therefore have a larger noise resistance; smaller coils have a lower noise resistance but, in turn, restrict the field of view to a smaller region.

It is highly desirable to extend the field-of-view by providing a set of surface coils arrayed with overlapping fields-of-view. However, it is desirable to at the same time maintain the high SNR of the single surface coil, if at all possible; this requires that mutual coil interactions be alleviated. Specifically, when obtaining signals from two or more coils simultaneously, it is important for the noise voltages in each coil to be as uncorrelated as possible. There will be some unavoidable noise correlation if the coils share a common noise source. But there can be unnecessary noise correlations if the noise currents in one coil induce voltages in other coils. It is also highly desirable to be able to construct a single optimal image, which maximizes the signal-to-noise ratio in each pixel of the composite single image, from the partial-image data of each of the plurality of surface coils in the array.

GB-A-2159626 discloses a nuclear magnetic resonance spectrometer wherein a plurality of mutually shielded transmitter/receiver coils are arranged around a sample tube which contains a gas to be tested. The coils are energised sequentially to derive a frequency spectrum. The arrangement is not intended for providing an image of a non-homogeneous sample such as the region of an anatomical sample.

US-A-4707664, as discussed above, discloses coils which are "remote coils" and are not disposed adjacent a patient.

### Brief Summary of the Invention

In one aspect the invention provides a method for receiving a different NMR response signal from each of a plurality of N surface coils (10") to produce an image of a sample including the steps of: (a) providing a two-dimensional array (10") of the plurality N of surface coils, with the array defining an image volume, with each surface coil being a single-turn surface coiled substantially identical to any other surface coil of the array and with each surface coil being closely-spaced to and having an overlapping field of view with, but having substantially no inductively coupling interaction with, at least all adjacent surface coils; (b) substantially simultaneously receiving at each one of the plurality N of surface coils a different one of a like plurality N of NMR response signals from an associated portion of the sample; (c) constructing each different one of a like plurality N of NMR images from the NMR response signals received by an associated surface coil; and (d) combining the plurality N of different images by weighting the signal intensity of each pixel of each image on a pixel-to-pixel basis by a factor determinedly the magnitude of correlated and uncorrelated noise between each adjacent and non-adjacent pair of surface coils to produce a single final NMR image of all sample portions from which a NMR response signal was received by any one of the surface coils.

In another aspect the invention provides an apparatus for receiving a different NMR response signal from each of a plurality of N surface coils (10") to produce an image of a sample including: (a) means for positioning a two-dimensional array (10") of the plurality N of surface coils, with the array defining an image volume, with each surface coil being a single-turn surface coil substantially identical to any other surface coil of the array and with each surface coil being closely-spaced to and having an overlapping field of view with, but having substantially no inductively coupling interaction with, at least all adjacent surface coils; (b) means for substantially simultaneously receiving at each one of the plurality N of surface coils a different one of a like plurality N of NMR response signals from an associated portion of the sample; (c) means for constructing each different one of a like plurality N of NMR images from the NMR response signals received by an associated surface coil; and (d) means for combining the plurality N of different images by weighting the signal intensity of each pixel of each image on a pixel-to-pixel basis by a factor determined by the magnitude of correlated and uncorrelated noise between each adjacent and non-adjacent pair of surface coils to produce a single final NMR image of all sample portions from which a NMR response signal was received by any one of the surface coils.

In a presently preferred embodiment of the present invention, each surface coil is connected to the input of an associated one of a like plurality of low-input-impedance preamplifiers, which minimize the interaction between any surface coil and other surface coils not immediately adjacent thereto. Surface coils of several geometries, e.g., circular, square and the like, can be utilized.

As preferred, a nuclear magnetic resonance (NMR) signal acquisition apparatus according to the invention includes a first coil, a first matching means, and a first preamplifier. The first coil has a source impedance $R_s$. The first preamplifier has an optimum source impedance $R_{opt}$ and an input impedance $R_{in}$, with $R_{in}$ being less than $R_{opt}$. The first matching means connects the first coil to the first preamplifier such that the source impedance of the first coil $R_S$ is transformed by the matching means to be approximately equal to the optimum source impedance $R_{opt}$ at the point of connection between the first matching means and the first preamplifier. At the same time, the low input impedance of the first preamplifier is transformed by the first matching means to be a value higher than the optimum source impedance $R_{opt}$ at the point of connection between the first matching means and the first coil.

The present invention preferably reduces the re-radiation of noise current circulating in a resonant NMR receiver coil. Without such attenuation, noise in one coil can be coupled into other coils in the system, thereby reducing the noise performance of the system. In this invention, the first matching means provides an impedance connected across the first coil which is a transformed value of the preamplifier input impedance $R_{in}$. That impedance is necessarily higher than the optimum source impedance $R_{opt}$, so that circulating currents, particularly noise currents, in the first coil are reduced.

As preferred the input impedance itself of the first preamplifier participates in the manipulation of the impedance presented to the first coil. It is thereby possible presented to the first coil. It is thereby possible to maintain optimum noise performance while at the same time reducing noise currents in the first coil. The first matching means may comprise a transmission line segment having a length approximately equal to one quarter of a wavelength at the NMR signal frequency. In order to match the source impedance $R_S$ to the optimum source impedance $R_{opt}$, the quarter-wave- length transmission line segment may have a characteristic impedance $Z_o$ determined by the equation:

$$Z_o = \sqrt{R_S R_{opt}}.$$

In another aspect of the invention, the preamplifier may include an input stage comprising an inductor-capacitor (L-C) network for establishing the input impedance of the preamplifier at the NMR signal frequency. The ratio of the optimum source impedance $R_{opt}$ to the input impedance $R_{in}$ may be greater than 20.

The invention preferably provides for clamping of large voltages encountered during a transmit phase of the NMR signal acquisition. The apparatus of this invention may include at least one half-wavelength transmission line segment interposed between the first matching means and the first preamplifier, and clamping means attached to at least one end of the half-wavelength transmission line segment. The clamping means thereby provides the desired attenuation. The half-wavelength transmission line segment has a characteristic impedance approximately equal to the optimum source impedance $R_{opt}$ to maintain optimum matching effectiveness for noise reduction purposes.

This invention provides an array of coils for simultaneous acquisition of multiple images. In that case, each coil is connected to a matching means and preamplifier similar to those according to this invention described above. Then, all of the coils in the array enjoy reduced noise currents due to the high impedance presented by each respective matching means to the associated coil, and noise coupling between the coils is consequently reduced.

These and other objects of the present invention will become apparent upon a reading of the following detailed description, when considered in conjunction with the drawings.

Brief Description of the Drawings

Figures 1 and 1a are, respectively, a schematic plan view of a single circular surface coil and a graph of the surface coil sensitivity S vs. frequency f response curve thereof;

Figures 1b and 1c are, respectively, a plan view of a pair of coupled circular surface coils and a graph of the sensitivity S vs. frequency f response curve thereof;

Figures 2a and 2b are plan views of pairs of surface coils, respectively of circular and rectangular shape, so situated so as to have substantially minimized interaction with one another;

Figure 3 is a plan view of a multiplicity of circular surface coils, illustrating the manner of two-dimensional placement thereof for minimized coupling of any two adjacent surface coils in the array thus formed;

Figure 4 is a schematic block diagram of an array of four substantially square, overlapped surface coils and of a like number of preamplifiers for use therewith;

Figure 4a is a plan view of a square surface coil, utilized in a multiple array thereof, in accordance with the principles of the present invention, and illustrating several dimensions and other features thereof;

Figure 5 is a schematic diagram illustrating the coupling between a pair of the surface coils of Figure 4, along with the preamplifier utilized with one of those surface coils, and useful in understanding principles of the present invention;

Figure 6 is a schematic diagram illustrating the manner in which a plurality of surface coils may be coupled one to the other for correlated-noise calculation purposes;

Figures 7 and 8 are, respectively, sets of various configurations of at least one surface coil, and a graph of the sensitivity S vs. frequency f response curves thereof;

Figures 9a and 9b are schematic diagrams illustrating various methods for coupling surface coils to an associated preamplifier;

Figure 10 is a schematic block diagram of apparatus for coupling the outputs of an array of a plurality of surface coils to quadrature-phase inputs of an NMR receiver, for imaging use.

Figure 11 is a perspective view of a nuclear magnetic resonance (NMR) signal acquisition apparatus according to the present invention;

Figure 12 is a schematic diagram for the PREDAMP circuit which forms a part of the apparatus of Figure 11; and

Figure 13 is a schematic diagram of an alternate construction for the PREDAMP circuit of Figure 11.

Detailed Description of the Invention

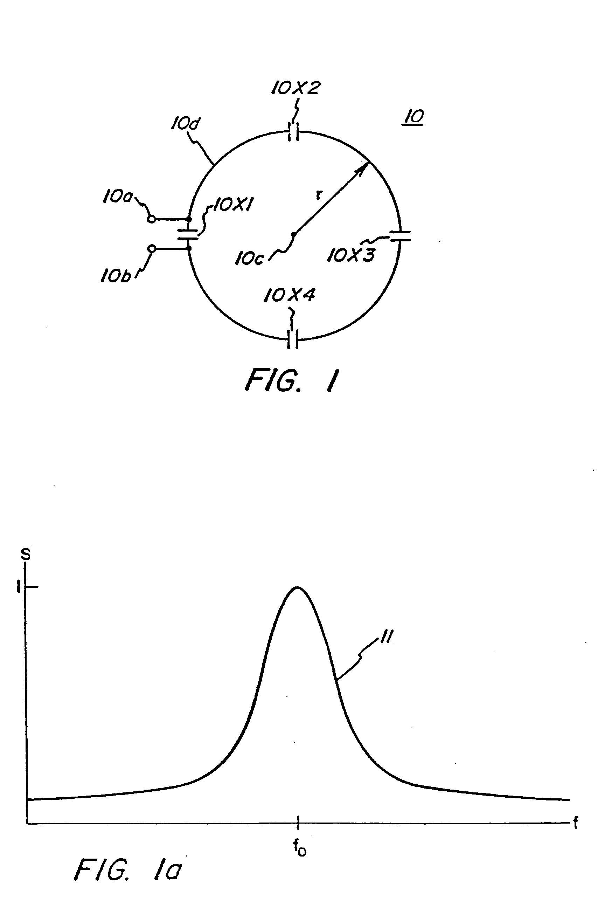

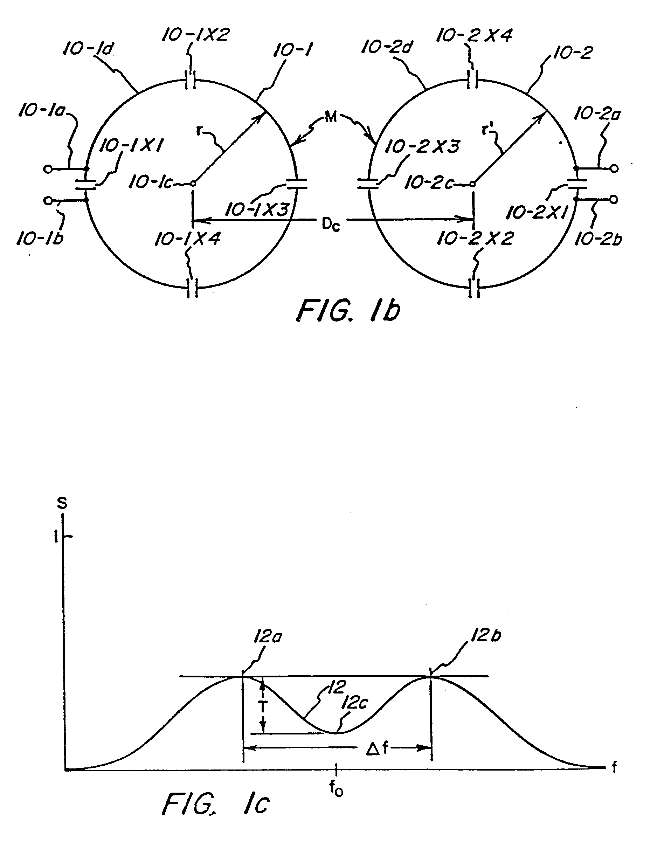

Referring initially to Figures 1 and 1a, a surface coil 10 extends from a first end 10a to a second end 10b, and is of substantially circular form, with a radius r about a center 10c. A conductive coil element 10d is in series connection with a plurality of impedance elements 10x, here electrical capacitances 10x1-10x4. When radio-frequency (RF) energy with substantially constant amplitude over a broad frequency spectrum impinges upon surface coil 10, the amplitude of received energy signals provided between coil ends 10a and 10b has a sensitivity S vs. frequency f response curve 11, with a sensitivity peak at the self-resonant frequency $f_o$ of the surface coil 10.

Referring now to Figures 1b and 1c, a first surface coil 10-1 can be located in proximity to a second surface coil 10-2, with a degree of inductive coupling M between the surface coil conductors 10-1d and 10-2d. The simple resonance of a single coil changes to two resonant modes of the two coils one mode corresponds to currents in the two coils going in the same direction, and the other mode corresponds to oppositely directed currents. The resonant frequencies of these modes are split away from the original resonance, with one resonant frequency now above the original resonance and one below, giving the appearance of a "double bump" in the response curve. This shift is seen in the sensitivity S vs. frequency f response curve 12, wherein a first peak 12a appears below self-resonant frequency $f_o$, while a second peak 12b appears above self-resonant frequency $f_o$; a minimum trough 12c is now present at the self-resonant frequency $f_o$. The normalized amplitude of the peaks 12a and 12b is less than the normalized unit amplitude of a single surface coil peak. The depth T of trough 12c, with respect to the amplitude of off-resonant peaks 12a and 12b, as well as the peak 12a-peak 12b separation $\Delta f$, depends at least in part upon the inductive coupling M, and therefore upon the center-to-center distance $D_c$, between coil centers 10-1c and 10-2c.

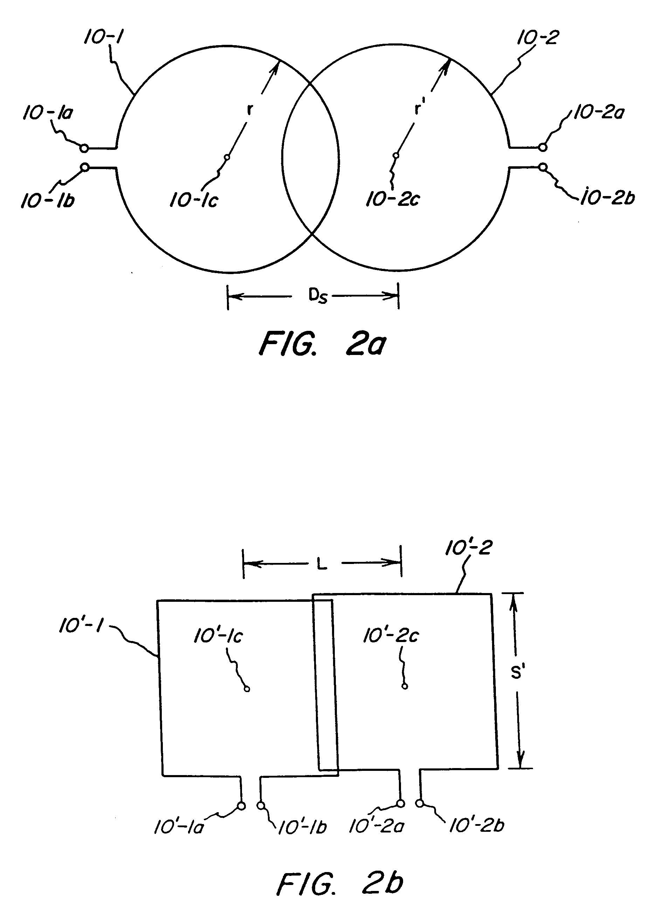

Referring now to Figures 2a and 2b, a coil-to-coil separation distance $D_s$, between the centers of a pair of adjacent coils in a one-dimensional array, can be found such that the mutual inductive coupling M is minimized. For a pair of circular surface coils 10-1 and 10-2 with substantially the same radii (r=r'), separation distance $D_s$ is approximately equal to 0.75d, or $D_s=1.5r$ (Figure 2a). Surface coils can be of non-circular form; a pair of non-circular coils 10'-1 and 10'-2 may be substantially square, with side dimension S'. For minimized mutual inductance M, the center 10'-1c of a first substantially-square coil is separated from the center 10'-2c of the adjacent substantially-square coil by coil-to-coil separation distance L of about 0.9 S'. It should be understood that mutual inductance can be reduced substantially to zero, thereby eliminating the mutual coupling between adjacent coils and the problem of resonance splitting with the nearest neighboring surface coils, but, because of slight imperfections in each practical coil, the approximate overlap only can be calculated from the above formulae and the exact amounts of overlap must be determined empirically. It will also be understood that additional slight differences in spacing and positioning may be required due to the placement of the gap between the coil ends, e.g., the gap between ends 10'-1a and 10'-1b of coil 10'-1; again, empirical fine adjustments of the spacing distance L (or distance $D_s$ for substantially circular coils) may be required if the gaps are changed from first and second positions, e.g., from opposite placement as shown in Figure 2a, to same-side placement as shown in Figure 2b.

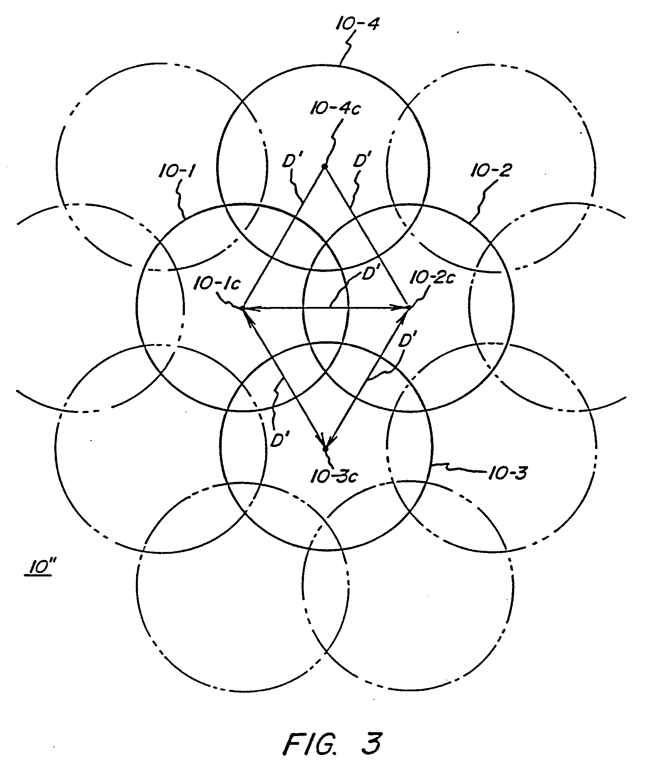

Referring now to Figure 3, the rules applied to the linear separation between a pair of adjacent surface coils in a one-dimensional array can also be applied to the center-to-center spacing D' of a plurality of adjacent surface coils forming a part of a two-dimensional array. Here, the three mutually-adjacent circular surface coils 10-1, 10-2 and 10-3 are arranged such that their centers 10-1c, 10-2c and 10-3c are each at a different apex of an equilateral triangle of side D', where the separation distance D' is about 1.5 times the radius of each of the surface coils. Similarly, the three mutually-adjacent circular surface coils 10-1, 10-2 and 10-4 have their centers 10-1c, 10-2c and 10-4c each at a different apex of an equilateral triangle of side D'. Additional substantially circular surface coils can be added, as shown by the chain-line circles forming phantom portions of the array; each added surface coil is positioned with its center at the apex of another equilateral triangle, formed with centers of real or imagined surface coils of the array. It will be seen that a three-dimensional array of surface coils can also be used, with pyramidal, cubical and the like subsets, or can be obtained by "wrapping" at least one two-dimensional sheet array over a three-dimensional object (such as a portion of human anatomy and the like) to enclose the sample object within the volume enclosed by the array "surface".

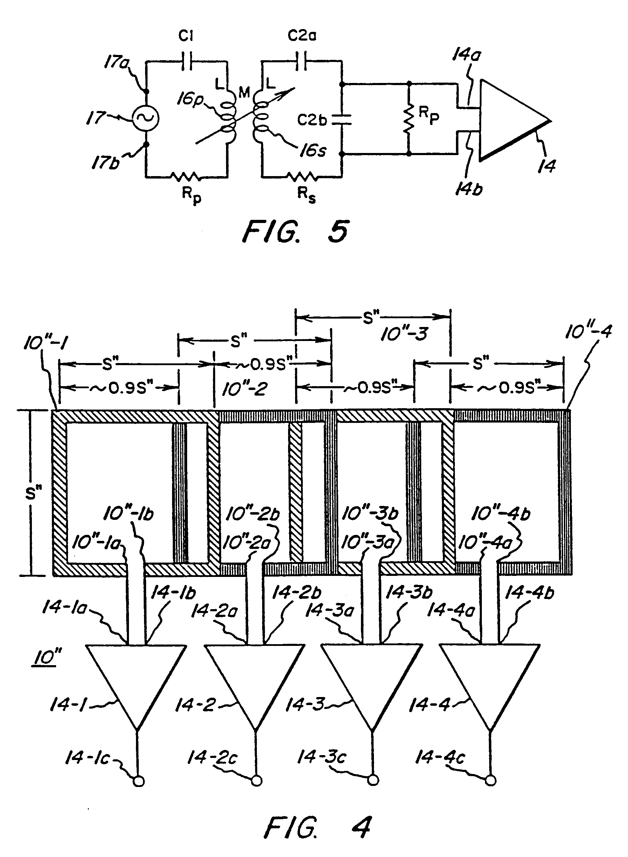

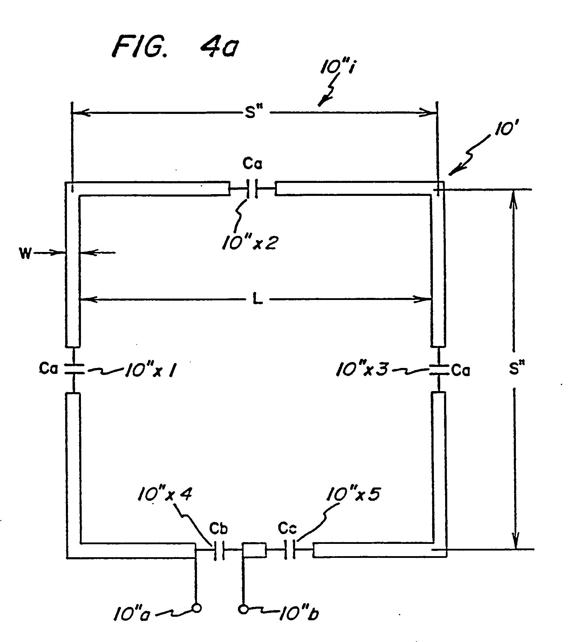

The interaction between next-to-nearest-neighbor coils, such as coil 10-4 interacting with coil 10-3, or interaction with even more distant coils, can be reduced to negligible levels by connection of each surface coil output to a preamplifier having a relatively low input impedance (typically, less than 10 ohms). This coil array-preamplifier set combination is illustrated in Figure 4, where a four-coil array 10", specifically utilized in a full-body NMR imager for imaging the human spine, includes first through fourth substantially square surface coils 10"-1 through 10"-4, each having a side dimension S" and an overlap distance of about 0.1 S' for substantially eliminating mutual coupling interaction with the adjacent coil(s). The ends 10"-ia and 10"-ib, where $1 \leq i \leq 4$, of each of the surface coils are connected to the associated inputs 12-1a and 14-1b of one of a like number of low-impedance RF preamplifier means 14. Thus, first surface coil opposite ends 10"-1a and 10"-1b are respectively connected to respective inputs 14-1a and 14-1b of first preamplifier 14-1, which provides a first preamplified surface coil signal at output 14-1c; the second surface coil 10"-2 has first and second ends 10"-2a and 10"-2b respectively connected to respective inputs 14-2a and 14-2b of the second preamplifier means 14-2, which provides a preamplified second surface coil signal at output 14-2c; the third surface coil 10"-3 has first and third ends 10"-3a and 10"-3b respectively connected to respective inputs 14-3a and 14-3b of the third preamplifier means 14-3, which provides a preamplified third surface coil signal at output 14-3c; and the fourth surface coil 10"-4 has first and second ends 10"-4a and 14-4b and 10"-4b respectively connected to respective inputs 14-4a and 14-4b of the fourth amplifier means 14-4, which provides a preamplified fourth surface coil signal at output 14-4c.

Each of the surface coils 10"-1 through 10"-4 can be fabricated, as shown by the surface coil 10"-i in Figure 4a, with conductive tape of width w (of about 0.5 inches (1.27cm) in this example) and with interior spacing L (of about 4.25 inches (10.8cm) in this example) so that the mid-side to mid-side spacing S" is here about 4.5 inches (10.8cm). Each leg is separated, at approximately the mid point thereof, by a lumped capacitance element. In the three legs not broken for coil connection, these lumped capacitance elements 10"x1, 10"x2 and 10"x3 have substantially identical capacitive values Ca (about 91 pF. for a coil utilized for $^1$H imaging in a system having a static magnetic field $B_0$ of about 1.5 Tesla, and a Larmor, or resonant, frequency $f_0$ of about 63.9 MHz.). The surface coil side having end connections 10"a and 10"b has a terminal-bridging capacitance element 10"x4 of another capacitive value Cb (about 150 pF.), and a side capacitance element 10"x5 of a third value Cc (about 300 pF.).

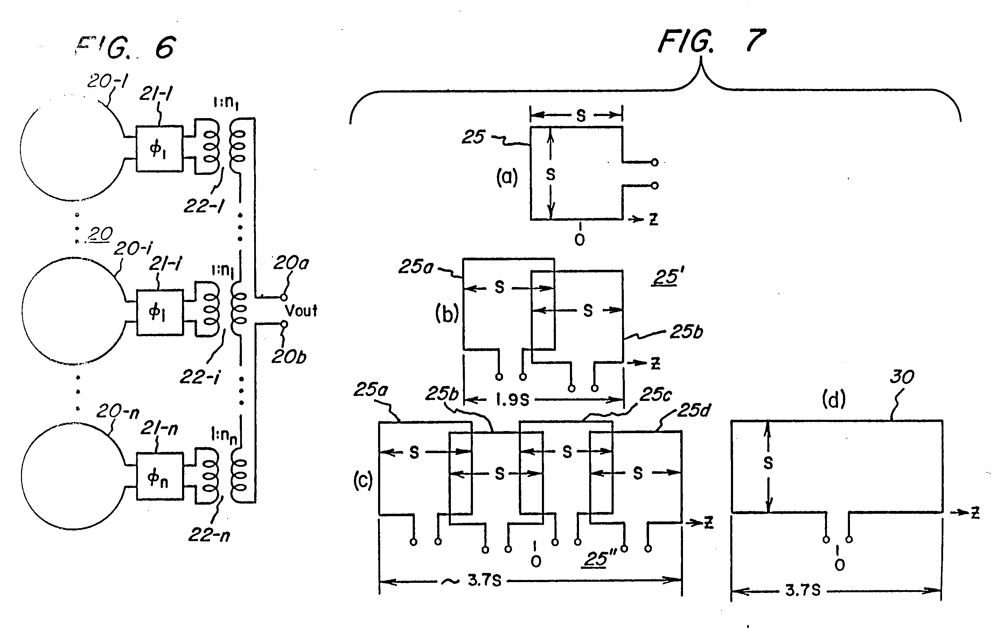

Referring now to Figure 5, the effect of connecting a low-input-impedance preamplifier to a next-nearest-neighbor surface coil can be analyzed by considering the two surface coils as being the primary winding 16p and the secondary winding 16s of a transformer 16. A coupling coefficient k exists between windings 16p and 16s. The primary and secondary windings are both considered to have the same inductance L, with a mutual coupling M=kL. The residual resistance $R_p$ or $R_s$ of the two windings is substantially similar, so that $R_p = R_s = R1$. If the first surface coil (winding 16p of inductance L, with a series capacitance C1 and series resistance R1) is driven by a source 17, we may remove the source and determine (a) whether the impedance seen across the source terminals 17a and 17b is substantially changed, and (b) whether additional dissipation and noise is introduced by the presence of the second surface coil (symbolized by winding 16s, another resistance R1 and the pair of surface coil capacitors C2a and C2b). Without the second surface coil present, the impedance of the primary loop is simply $R_1 = R_P$. When the second surface coil is added, the impedance $Z_A$, between terminals 17a-17b, is given as

$$Z_A = R1 + \omega^2 M^2 / Z_s \qquad (1)$$

where $Z_s$ is the series impedance of the secondary surface coil loop and $\omega$ is the resonant frequency. The secondary loop is tuned with multiple capacitors, which can be reduced to capacitors C2a and C2b; the input impedance $R_p$ of a low-impedance preamplifier 14 is effectively in parallel across the second loop output capacitance C2b. Generally speaking, this output capacitance is much larger than the net coil series capacitance C2a, so that we can quantify the capacitance relationship as $C2b = (N-1) \cdot C2a$. Therefore, the net series capacitance of the secondary surface coil circuit is about C2b/N. The exact impedance $Z_A$ between 17a-17b can thus be calculated as

$$Z_A = R1 [1+k^2 Q^2/(1+[Q/N](X_c/(R_p-jX_c)))] \tag{2}$$

where the resistance in each of the primary and secondary circuits is approximately R1, the circuit quality factor $Q= L/R1$ and $X_c$ is the reactance of capacitor C2b.

We have found it useful to look at two limits. The first limit occurs if a preamplifier is not attached to capacitor C2b and the second loop is resonant; here the preamplifier impedance $R_p$ goes to infinity and

$$Z_A = R1 (1 + k^2 Q^2) \tag{3}$$

Although k is small, the quality factor Q could be large, and the $k^2 Q^2$ term in equation (3) might be significant. At the second limit, the preamplifier input impedance $R_p = 0$ and equation (2) reduces to

$$Z_A = R1 (1-jk^2 QN) \tag{4}$$

Comparing equations (3) and (4), it will be seen that the effect of the second loop impedance on the first loop can be reduced by a factor of (Q/N), which is a factor substantially greater than 1, by making the preamplifier input impedance substantially equal to zero, as compared to a resonant second loop. The reflected impedance is now imaginary and, at worst, produces a first loop frequency shift, instead of an apparent additional loss in the primary loop. For analysis of a real situation, the illustrative square coil of Figure 4a (utilized in a four-coil spinal imaging array at 64MHz), has parameters of: N=6.45, $X_c$=16.6 ohms, Q =361 unloaded and Q=24 loaded. The following Table contains values for the impedance reflected back into the primary surface coil, from the secondary surface coil, when a preamplifier is placed across the secondary surface coil terminals. The Table entries are given in terms of ratio of the total impedance $Z_A$, seen at the primary coil terminal 17a/17b, to the loss resistance R1 which would normally be seen if only the resonant primary surface coil were present. Thus, the impedance ratio has both a real component and an imaginary component. It is the real component which indicates the relative increase in noise, that is, a ratio, for example, of 1.07 (for the unloaded coil with a 10 ohm preamplifier attached to the secondary coil) indicates that the resistance, and hence the noise power, in the primary surface coil has increased by 7% because of the presence of the second coil. Therefore, the system noise figure has increased by about 0.3db, which is a relatively small amount. It will be seen that in none of the loaded coil examples is there a significant increase in noise, even when the preamplifier is not attached to the second surface coil. The only significant effects occur for unloaded surface coils, with either a 50 ohm input impedance preamplifier, or with no preamplifier present. The imaginary portions of the impedance correspond to a small shift of resonant frequency, which is also seen to be relatively insignificant, as all of the imaginary impedance ratio portions have, at most, (R1/10), that is no more than 10% of the series loss resistance R1, and therefore must limit the total frequency shift to less than 0.1 of the resonance width.

## TABLE OF $Z_A/R1$

| Coil Config. | $R_p = 0 \, \Omega$ | $5 \, \Omega$ | $10 \, \Omega$ | $50 \, \Omega$ | $\infty \Omega$ ohms |
|---|---|---|---|---|---|
| Unloaded, Q=361 | 1.00-j 0.114 | 1.04-j 0.113 | 1.07-j 0.112 | 1.33-j 0.103 | 1.38 |
| Loaded, Q=24 | 1.00-j 0.007 | 1.00-j 0.006 | 1.01-j 0.005 | 1.01-j 0.002 | 1.03 |
| for k = 0.007 | $X_c$=15.4 ohms | and N = 6.45 | | | |

We have also calculated the magnitude of current generated in the coil of interest due to currents flowing in a remote coil. The current in the first coil, due to voltages in the first and second coils, is given by:

$$I1 = (-j\omega MV2-V1Z2)/(Z1Z2+\omega^2 M^2) \tag{5}$$

where V1 is the first surface coil voltage and Z1 and Z2 are respective first and second surface coil impedances. The ratio of current in coil 1 caused by the second coil voltage 2 to the current caused by voltage 1 is

$$I1(2)/I1(1) = (j\omega M/Z2)(V2/V1) = (j\omega Lk/Z2)(V2/V1) \tag{6}$$

Substitution of the second surface coil impedance yields

$$I1(2)/I1(1)=(V2/V1)(jkQ)/(1+(Q/N)[jX_c/(X_c+jR_p)])$$ (7)

for a preamplifier input impedance $R_p$ comparable to, or smaller than, the capacitive reactance $X_c$, this reduces to

$$I1(2)/I1(1) \; kN=[X_c+jR_p)/X_c](V2/V1)$$ (8a)

$$I1(2)/I1(1) = kN[1+(jR_p/X_c)](V2/V1)$$ (8b)

utilizing k=0.007 and N=6.45, this reduces to

$$I1(2)/I1(1) = 0.045$$ (9)

so that the signal appearing in surface coil 1, due to a surface coil 2 signal, is decreased by a factor of 22 relative to the signal in surface coil 2; that is, a surface coil 2 signal is over 22 times less strong in surface coil 1 than in surface coil 2.

Individual images from each of a plurality of surface coils are useful, although the construction of a single composite image from the overlapping fields-of-view of an array of a plurality N of surface coils is highly desirable. If properly constructed, such a composite image will include quadrature components and will have a maximum possible signal-to-noise ratio (SNR). The amount of coil overlap required to substantially eliminate adjacent coil mutual inductance is substantially that amount of overlap which also yields a significant field-of-view overlap, allowing a single composite image to be constructed with a minimal SNR variation. The increased SNR, if realized at all, requires that the images from the various surface coils be combined on a point-by-point basis, utilizing a detailed knowledge of the magnetic fields of all of the surface coils, as well as of the correlated and uncorrelated noise between surface coils. The general sensitivity of a single coil is proportional to the transverse magnetic field that the coil creates at any point, for a given current; the coil noise voltage is proportional to the square root of the coil terminal resistance. The SNR at a volume element (voxel) located at a point (x,y,z) is

$$SNR = \omega MVB_t(x,y,z)/(4KTR)^{1/2}$$ (10)

where $B_t(x,y,z)$ is the transverse magnetic field created by the surface coil at the point (x,y,z) for a unit current flowing in that surface coil, M is the magnetization per unit volume, V is the voxel volume, $\omega$ is the resonant frequency and R is the noise resistance $R_n$, which can be expressed in terms of a time average integral over the ohmic losses, such that

$$R_n = \langle (1/\sigma)\int \bar{J} \cdot \bar{J} \; d^3v \rangle$$ (11)

where $\bar{J}$ is the induced current density in the sample for unit current in the coil and $\sigma$ is the sample conductivity. If the induced current is expressed in terms of the electric field, noise resistance $R_n$ becomes

$$R_n = \langle \sigma \int \bar{E} \cdot \bar{E} d^3v \rangle$$ (12)

where $\bar{E}$ is the electric field produced for a unit current in the surface coil.

As shown in Figure 6, a plurality N of surface coils 20-I through 20-n, of an array 20, can have their output voltages phase shifted, utilizing a like plurality N of phase shifters 21, and linearly combined, utilized a like plurality N of transformers 22, to form a single output $V_{out}$ between array terminals 20a and 20b. Each of the transformers, considered to be an ideal lossless transformer, has a turns ratio $I:n_i$, for the i-th transformer 22-i, where $I \leq i \leq N$. Each coil has an instantaneous output voltage $v_i(t)$ given by

$$v_i(t) = \omega MVB_t(x,y,z)_i cos(\omega t+\psi_m+\theta_i)$$ (13)

where $\theta_i$ is the angle of the RF magnetic field measured from some fixed reference in the laboratory frame and $\psi_m$ is an arbitrary phase of the rotating nuclei. The combined signal $V_{out}$ from the surface coil array 20, due to magnetization at some point (x,y,z) is

$$V_{tot} = \omega MV \sum_{i=1}^{N} n_i \, [B_t(x,y,z)]_i \cos(\omega t + \psi_m + \theta_i + \phi_i) \quad (14)$$

The peak amplitude of this voltage is the desired signal and is given by

$$|V_{tot}|^2 = (\omega M V)^2 [\sum_{i=1}^{N} n_i [B_t(x,y,z)]_i \cos(\omega t + \psi_m + \theta_i + \phi_i)^2 \\ + ([\sum_{i=1}^{N} n_i \, [B_t(x,y,z)]_i \sin(\omega t + \psi_m + \theta_i + \phi_i))^2] \quad (15)$$

As viewed from the array output terminals 20a-20b, the total noise resistance is obtained by injecting a current of unit amplitude into the transformer secondaries and taking the time average of the losses integrated over the volume of the sample (a human patient and the like) being imaged. If a current of unit amplitude, $\cos(\omega t)$, is injected into the secondary windings of series-connected transformers 22, a current (phase shifted by an amount $-\phi_i$ and of amplitude $n_i$) flows in any coil 20-i. The total electric field in the sample is then given by

$$\overline{E}_{tot} = \sum_{i=1}^{N} n_i \overline{E}_i \, \sin(\omega t - \phi_i) \quad (16)$$

where we have separated out the spatial dependence of the electric field, given by $\overline{E}_i$, and the time dependence. Combining equations (12) and (16), changing the order of integration and summation and averaging over time, the loss resistance, as seen from the transformer secondary, can be found as

$$R_{tot} = \sum_{i=1}^{N} \sum_{j=1}^{N} n_i \, n_j \, R_{i,j} \quad (17)$$

where we have defined the mutual noise resistance

$$R_{i,j} = (\sigma/2) \int \overline{E}_i \cdot \overline{E}_j \, d^3v \cos(\phi_i - \phi_j) \quad (18)$$

The noise resistance matrix will therefore contain all of the information about the correlated and uncorrelated noise between surface coils. That is, $R_{i,j}$, where i=j, is the noise resistance of coil 20i, held in isolation; however, noise resistance $R_{i,j}$, where i is not equal to j, is the added resistance when coil 20i and coil 20j are used in combination, therefore representing the correlated noise between surface coils 20i and 20j. Note that the noise is completely uncorrelated if the electric fields are phase shifted by 90 degrees. The signal-to-noise ratio, at point (x,y,z) of the combined surface coil set is therefore

$$SNR = |V_{tot}|/\sqrt{4KTR_{tot}} \qquad (19)$$

where $V_{tot}$ and $R_{tot}$ are given by Equations (15) and (17). The optimum phase shifts are determined by taking the partial differential $\partial SNR/\partial\phi_i$ for each value of i and equating the result to zero. We find that the resultant set of equations are satisfied if $\phi_i = -\phi_i$. Thus the optimum values to assign to the external phase shifts exactly cancel the phase shift of the induced NMR voltage. Substituting this value of $\phi_i$ into $V_{tot}$ of Equation (19) we obtain

$$SNR = \omega MV \left( \sum_{i=1}^{N} n_i [B_t(x,y,z)]_i \right) / (4KTR_{tot})^{1/2} \qquad (20)$$

This signal-to-noise equation applies equally as well to any method utilized for image summation, even though derived with the coils connected through a series of lossless phase-shifters and transformers. When the signals are combined to obtain a single optimum image, the turns ratios are interpreted, and computed, as the relative weights for combining each surface coil data in the combined image. Thus, we must choose the relative weights ($n_i$) of the surface coils so as to maximize Equation (20) on a point-by-point basis if an improved SNR is to be realized at all points in an image. Equivalently, by minimizing the total noise resistance while holding the combined signal level constant, the SNR is maximized. We therefore want to minimize a function F where

$$F = \sum_{i=1}^{N} \sum_{j=1}^{N} n_i n_j R_{i,j} + \lambda \sum_{i=1}^{N} n_i [B_t(x,y,z)]_i \qquad (21)$$

where $\lambda$ is a Lagrange multiplier of the constraint on the signal level. Values $n_i$ which minimize Equation (21) are obtained by setting the partial derivative of F, with respect to value $n_i$, to zero for each value $n_i$. The result is given by $\underline{n}$, where

$$\underline{n} = -\lambda \, \underline{R}^{-1} \underline{B}(x,y,z) \qquad (22)$$

where $R^{-1}$ is the inverse of the noise resistance matrix $[R_n]$, $\underline{B}(x,y,z)$ is a vector containing the magnitudes of the transverse field created at point (x,y,z) by the surface coils, and $\underline{n}$ is a vector containing the optimum turns ratios, or weights. The exact value of $\lambda$ is relatively unimportant, since it will cancel out when the optimum weights solution of Equation (22) is substituted into Equation (20), to obtain the optimum SNR value. That is, the value of $\lambda$ is important only to scale the pixel intensities of the final composite image. It will be seen that the optimum weighting function, or set, is in general a function of pixel position. Thus, the NMR signal from each of the coils must be acquired separately and would only allow maximization, at best, of the SNR at a single point, if all of the severally acquired signals were added in some manner externally. Therefore, after each separate acquisition, a separate image should be reconstructed for each surface coil and then a single combined image must be constructed by adding the individual image results on a pixel-by-pixel basis, in accordance with Equation (22). Although there is a phase shift in the instantaneous NMR signal in the coils, due to the different directions of the RF magnetic fields in the coils, forming a single image by combining weighted magnitude images is still correct. The phase shift is taken into account in the noise resistance matrix, as given by Equation (18) with $\phi_i = -\theta_i$, where $\theta_i$ is the direction of the magnetic field at position (x,y,z). It will be seen that the noise resistance matrix values can be based on measurement or calculation, although measured values are preferable, since actual measured values will take into account the small losses of the surface coil and any associated protective circuitry; these losses only show up as contributing to diagonal terms in the noise resistance matrix, as such losses exhibit no correlation between coils. The computed matrix for a four-coil array 10" (Figure 4) is derived from the matrices of calculated mutual resistance $[R_n]$ (calculated from Equation (18), with coil currents in phase), via Equation (20), and

mutual inductance [$L_m$], with the noise resistance matrix corresponding to sample losses with the coils placed against an infinite half plane of conducting material.

MATRICES

$$\underline{R}_n = \begin{bmatrix} 1 & 0.40 & 0.147 & 0.078 \\ 0.40 & 1 & 0.40 & 0.147 \\ 0.147 & 0.40 & 1 & 0.40 \\ 0.078 & 0.147 & 0.40 & 1 \end{bmatrix}$$

$$\underline{L}_m = \begin{bmatrix} 1 & 0 & 0.007 & 0.0016 \\ 0 & 1 & 0 & 0.007 \\ 0.007 & 0 & 1 & 0 \\ 0.0016 & 0.007 & 0 & 1 \end{bmatrix}$$

It should be noted that, although the coil overlap forces the mutual inductance between adjacent coils to zero, there is still significant correlated noise indicated by the non-zero off-diagonal elements of the noise resistance matrix.

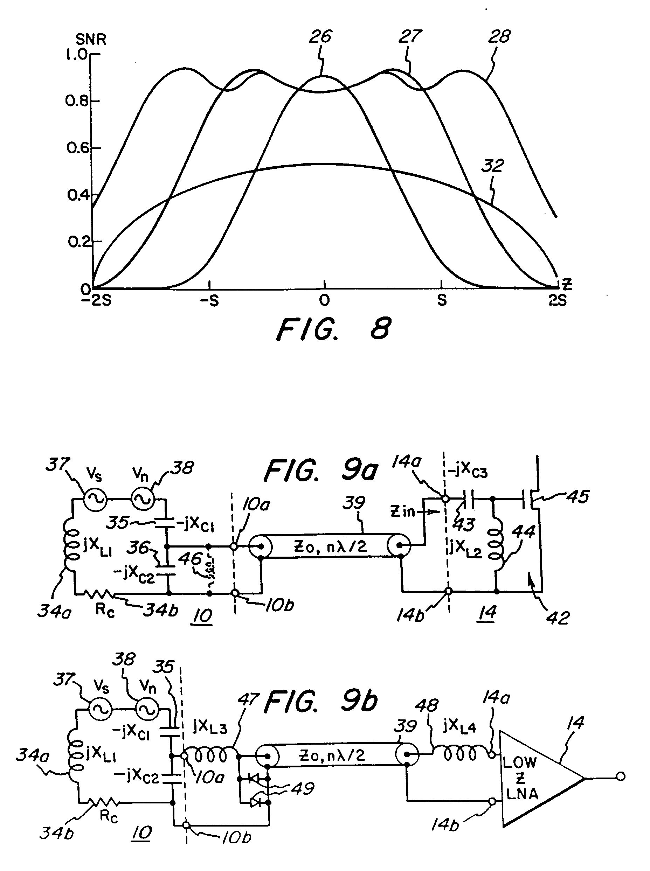

Referring now to Figures 7 and 8, the computed signal-to-noise ratio for a number of surface coil arrays are compared. In case (a) of Figure 7, a single substantially-square surface coil 25 is so positioned about the zero center of a uniform object as to provide a SNR curve 26 (Figure 8) having a normalized signal-to-noise ratio of 1.0 at Z=0. An array 25' of a pair of the substantially-square surface coils 25a and 25b, separated by the overlapped distance for substantially zero interaction between adjacent surface coils, has a sensitivity vs. position curve 27 (Figure 8), with a substantially constant SNR over the range from about Z=-S to about Z=-+S. An array 25" of four substantially square surface coils 25a-25d, arranged as shown in Figure 4, has a SNR vs. position curve 28, with a substantially constant signal-to-noise ratio over a range from about Z=-1.6S to about Z=+1.6S. In contrast, a single, rectangular surface coil 30, having a width equal to the side dimension S of array 25" and a length of about 3.6S, substantially equal to the overall length of array 25", has a SNR vs. position curve 32, with a maximum relative SNR value of only about 0.5 of the four-surface coil array 25". It will therefore be seen that the composite of a plurality of surface coils has almost the sensitivity of a single surface coil, with the field of view of a larger single coil (which by itself has a lower sensitivity).

We presently prefer to have the surface coils 10, which are within the high-magnetic-field region of the NMR system, located at considerable distance from the associated preamplifier means 14, which are not in the high magnetic field region. As seen in Figure 9a, the equivalent of surface coil 10 is an inductance 34a (with reactance $+jX_{L1}$) and a resistance 34b (of magnitude $R_c$) in series with tuning and matching capacitances 35 and 36, respectively, of reactance $-jX_{c1}$ and $-jX_{c2}$. The signal and noise voltages appearing across the surface coil are represented by the signal voltage $V_s$ source 37 and the noise voltage $V_n$ source 38. The surface coil terminals 10a and 10b are connected to a first end of a coaxial cable 39, having a length equal to an integral number of half-wavelengths at the NMR resonance frequency in use; the opposite cable end is connected to input terminals 14a and 14b of the associated preamplifier. The surface coil is tuned to present a noise resistance, between terminals 10a and 10b, which is equal, under loaded conditions, to the characteristic impedance $Z_0$ of cable 39, so that the cable adds a minimized amount of noise energy. Preamplifier 14 contains a series- resonant noise-matching network 42, comprised of a capacitor 43, (of reactance $-jX_{c3}$) and an inductance 44 (of reactance $+jX_{L2}$), which transforms the noise resistance to that resistance required to noise-match the input of a preamplifier active device 45, such as a GaAsFET and the like. The preamplifier, having a very low noise figure, low input impedance and relatively wide bandwidth can have its input capacitor 43 substantially mistuned without substantially degrading the preamplifier noise figure at a given frequency. Thus, the input impedance $Z_{in}$ of preamplifier 14 which is normally resistive and of a fairly low value (less than about 5 ohms), when tuned, can by proper adjustment of capacitor 43 be made to appear inductive, with a reactance $+jX_{c2}$. By thus retuning the preamplifier to be inductive, the preamplifier input impedance $Z_{in}$ at the preamplifier end of the cable also appears as an inductive reactance 46 (shown in broken line) at the surface coil end of cable 39, and resonates with the surface coil output capacitor 36 to form a parallel resonant blocking circuit which is active during reception to minimize current flowing in surface coil 10 and thus prevent noise (and signal) received by one surface coil from being coupled to other coils through their mutual inductances. Therefore, by causing each preamplifier 14 and associated cable 39 to present a parallel resonating inductance to the associated surface coil, images taken with the surface coil array will maintain a high SNR and show substantially no evidence of coupling to other coils. Since the foregoing often requires that a specific cable 39 be used with a specific preamplifier 14, we prefer to tune the preamplifier input circuit 42 to resonance and utilize an external inductance 47 or 48 in series with the low input impedance preamplifier, as shown in Figure 9b. This external inductance (of reactance $+jX_{L4}$) can be either an inductance 47, connected between the surface coil terminal 10a and the input center conductor of coaxial cable 39, or a physically separate inductance 48 (of reactance $+jX_{L4}$) connected between the center of the coaxial cable 39 at it output end and the associated low-noise amplifier (LNA) input terminal 14a. A combination of the two inductances 47 and 48 can also be used, if desired. This preferred use of at least one external inductance allows the preamplifiers to be inter-changed without retuning and permits the inductor to be used in forming a blocking circuit during transmission, in conjunction with a pair of anti-parallel-connected protection diodes 49.

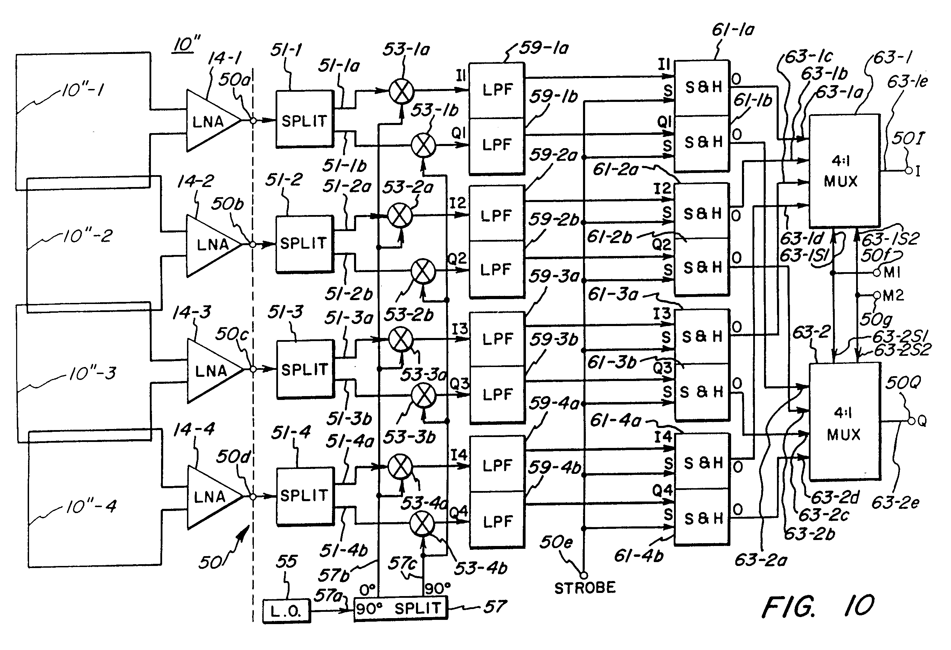

Referring now to Figure 10, a receiver front-end assembly 50 is shown for operating upon the plurality N of preamplified NMR response signals, equal in number to the number N of the surface coils in associated surface coil array 10", for generation into a pair of baseband quadrature signals for image processing and generation by means well known to the art. Thus, a surface array coil 10" has N=4 surface coils 10"-1 through 10"-4 and has associated low-noise amplifiers LNAs 14-1 through 14-4, each for providing a different preamplified reception signal to a different associated one of NMR receiver inputs 50a-50d. The single preamplified signal at one of inputs 50a-50d is applied to a signal input of an associated splitter means 51-1 through 51-4. Each splitter means provides a pair of in-phase signals at the pair of outputs 51-1a and 51-1b, 51-2a and 51-2b, 51-3a and 51-3b or 51-4a and 51-4b thereof. Each split signal is applied to the RF input of an associated one of a plurality (2N) of signal mixer means 53. A local oscillator means 55 provides a RF signal, at the Larmor resonance frequency of the molecule under study, to the input 57a of a quadrature signal splitting means 57. Each of a pair of quadrature-phase outputs 57b and 57c respectively provides a reference 0° signal and a quadrature 90° signal. The reference 0° signal at output 57b is coupled to the injection input of a first one of each pair of mixer means 53 connected to a like output of an associated one of splitters 51, while the quadrature 90° signal at splitter output 57c is connected to the injection input of the remaining plurality N of that mixer means. Thus, all of the "a" mixers, e.g., mixers 53-1a, 53-2a, 53-3a and 54-4a, receive the reference local oscillator signal, while the injection inputs of each of the "b" mixers, e.g., mixers 53-1b, 53-2b, 53-3b and 53-4b, receive the quadrature local oscillator signal. A baseband signal is provided at the output of each mixer means 53, with each of the "a" mixers, having received the reference 0° local oscillator signal, providing an in-phase I signal, and each of the "b" mixers, having received the quadrature 90° local oscillator signal, providing a quadrature-phase Q signal. Thus, in-phase baseband signals I1-I4 are each respectively provided by an associated one of mixers 53-1a through 53-4a, while each of the quadrature-phase signals Q1-Q4 is respectively provided at the output of an associated one of mixers 53-1b through 53-4b. Each of the 2N baseband signals is individually filtered in an associated one of low pass filter means 59, and the filtered signal is provided to the signal input of an associated one of a plurality 2N of sample-and-hold (S&H) means 61, each also having a sample S input receiving a strobe signal from a front end input 50e. Thus, while all of the sample S inputs are connected in parallel, each of the signal inputs of the S&H means receives a different baseband signal. That is: means 61-1 receives the filtered first in-phase I'1 signal; means 61-b receives the first quadrature-phase filtered signal Q'1; means 61-2a receives the second filtered in-phase I'2 signal; fourth means 61-2b receives the second quadrature-phase Q'2 signal, and so forth. The output O of each sample and hold means 61 is connected to an associated input port of one of another plurality M of K:1 multiplexing (MUX) means 61. The number M of MUX 63 is given by M=2N/K; here, with N=4 and K=4, M=2 means 63-1 and 63-2. The particular one of the K inputs 63-1a to 63-1d or 63-2a through 63-2d, of respective means 63-1 or 63-2, connected to the single means output 63-1e or 63-2e (and therefrom to the I output 50-i or the Q output 50-q of the front end means 50) is determined by the binary signal states of two multiplexing control signals M1 and M2, provided to the MUX means respective selection inputs 63-1s1 and 63-2s1, or 63-1s2 and 63-2s2, respectively, from front end means selection signal input terminals 50f or 50g, respectively.

In operation, front end means 50 continuously receives each of the plurality N of preamplified response signals, at the associated one of the plurality of N individual input terminals 50a. Each surface coil preamplified signal is split into two substantially equal-amplitude portions and is mixed with an in-phase or quadrature-phase local oscillator signal, and subsequently low pass filtered to provide continuous in-phase and quadrature-phase baseband signals from each surface coil antenna. The signals are all sampled, substantially at the same instant, by action of each of means S&H 61 responsive to the common strobe signal. The held analog amplitude value of each of the two N signals is then multiplexed, by means 63 operating at K times the strobe frequency, so that time-diversity-multiplexed I or Q analog signals are provided to the subsequent processing means (not shown). It will be understood that greater or lesser numbers of surface coils in the array, and similar number of channels, multiplexing ratios and the like, are contemplated. It will be understood that the strobe signal can be synchronized directly to the multiplexed selection signal, by means well known to the signal processing arts; the frequency and form of strobe signal(s) are selected to satisfy well known signal processing constraints.

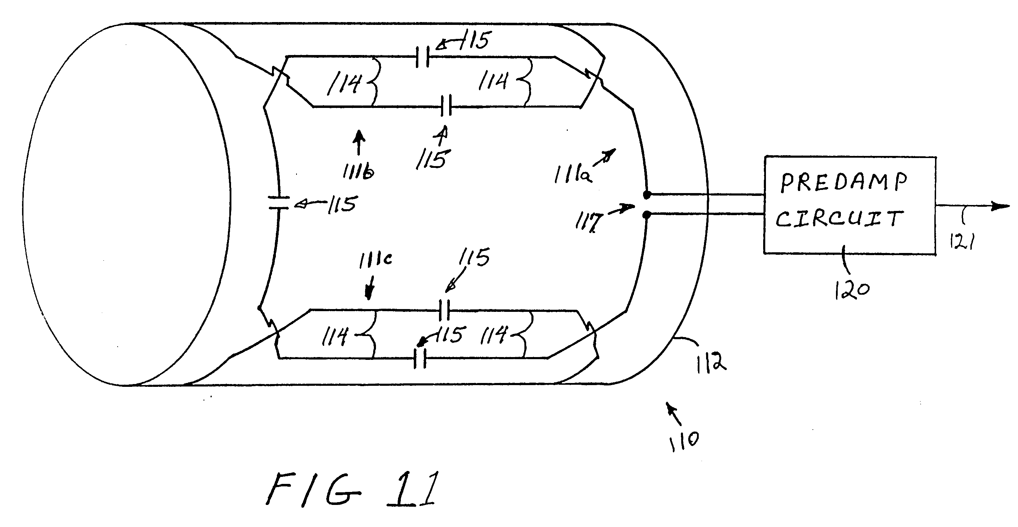

Referring to Figure 11, a coil assembly 110 comprises four individual coils 111a - 111d (only coils 111a - 111c are visible in Figure 11) placed on the surface of a cylindrical coil form 112. The coil form 112 is approximately 6 inches (15.24 cm) in diameter and 5 inches (12.7 cm) in length, and formed of a suitable insulating material. The coils 111a-111d are roughly square in shape and each coil 111a - 111d spans approximately 111° around the cylindrical form 112. As a result, the longitudinal edges of each of the coils 111a - 1111d overlaps with its next nearest neighboring coil. Each coil 111a - 111d includes conductors 114, functioning as inductive sections, in series with capacitors 115. Each coil 111a - 111d is thereby essentially a series L-C circuit. The conductors 114 are formed of 3/8 inch (0.953 cm) wide copper tape fastened onto the surface of the form 112. At the corners of each coil 111a - 111d, the conductors 114 are run diagonally so that the intersections between coils are at right angles. Where conductors 114 cross, one of the conductors is routed as a small rectangular bridge over the other conductor 114. Each coil 111a - 111d includes four conductors 14 separated by gaps. Three of the gaps are bridged by capacitors 115 soldered onto the copper tape. The other gap 117 is open, and serves as a connection point for processing circuitry described below.

Surface coil arrays are generally known in the art for use in NMR signal acquisition. A common problem in such coil arrays is the coupling of noise between coils in the array. This phenomenon occurs as noise currents as part of an incident signal on one coil can essentially "re-radiate", causing additional noise currents in a nearby mutually coupled coil. The mutual coupling between coils can be alleviated to some extent by their relative placement, for example orthogonally or with a certain degree of overlap. However, ideal placement of the respective coils is often mandated by a particular application, and cannot always be accomplished in an ideal manner. Further, one object of this invention to provide an enhanced composite image based on a novel combination of separate images obtained from multiple coils with overlapping fields of views, particularly when the coils in the array are not in the same plane. For example, the coil structure of Figure 11 is suitable for imaging limbs of a human subject, and mutual coupling between the coils 111a - 111d cannot be eliminated through placement alone.

In order to provide an even greater reduction of noise coupling between coils in an array, one aspect of this invention is to reduce the noise currents induced in each individual coil 111a - 111d, thereby greatly attenuating the re-radiation of that noise to other coils in the array. The circuit for providing that function is integrated into a preamplification stage for each coil 111a - 111d, and is referred to herein as a PREDAMP circuit 120. The PREDAMP circuit 120 is coupled to coil 111a across the gap 117, and amplifies the signal incident on the coil 111a in such a manner, described in detail below, that circulating currents, both signal and noise, in the coil 111a are minimized. The amplified signal is then output on line 121 for processing by the remainder of the NMR system. Although only one PREDAMP circuit 120 is shown in Figure 11 connected to coil 111a, it should be understood that each other coil 111b - 111d is also connected to its own PREDAMP circuit 120, thereby providing for simultaneous acquisition of signals 121 corresponding to all four coils 111a - 111d.

Referring to Figure 12, each coil 111a - 111d can be modeled as an equivalent series R-L-C circuit, hereinafter referred to as coil 124. The "L" element 125 of coil 124 corresponds to the inductance of the conductors 114 at the Larmor frequency of the sample of interest, approximately 64 MHz in this embodiment. The "C" element 127 corresponds to the capacitors 115 distributed across the gaps between conductors 114. And the "R" term of the coil 124 represents the net source impedance of the coil 124, which is predominantly due to the loading caused by the sample under study, for example, a human patient, which is usually a fairly lossy medium.

In prior coil-preamplifier combinations, the series coil elements were closed, usually by a capacitor (not shown), to provide an optimum impedance match for the transmission line and preamplifier used. It was, therefore, important in prior coils to maintain a fairly high quality factor, or "Q" to maximize the signal developed across the matching capacitor.

The PREDAMP circuit 120 operates in a substantially different manner by presenting a high impedance across the coil terminals 117, substantially lowering the Q of the coil 124. As a result, currents circulating in the coil 124 are suppressed, and re-radiation that would have been caused by those currents are likewise reduced.

Amplifiers typically used as NMR preamplifiers can be characterized by their input impedance, e.g. circuit loading, and an optimum source impedance. The optimum source impedance is used to describe that source impedance which provides the best noise performance, in terms of the signal-to-noise ratio (SNR), for driving the preamplifier 130. Driving the preamplifier 130 with an imbalanced source impedance, e.g. other than the optimum, results in degradation of the noise performance of the preamplifier 130. Therefore, one problem with lowering the Q of the coil 124 is that it must be done in such a way as match the optimum source impedance of the preamplifier 130. A second concern in lowering the Q of coil 124 is that it is not feasible to do so simply by adding resistance, either in series or parallel. The introduction of resistance at a non-absolute zero temperature would result in additional noise in itself, usually more than could be eliminated by lowering the coil Q. The problem of implementing a PREDAMP circuit is then twofold; (1) to present a high impedance to the coil terminals 117, while at the same time (2) presenting the optimum source impedance to the preamplifier 130. Both of these objectives are achieved in a PREDAMP circuit 120 according to this invention by utilizing the input impedance itself, of the preamplifier 130, as a part of a matching network for directly manipulating the Q of the coil 124.

Still referring to Figure 12, a PREDAMP circuit, according to this invention, includes a preamplifier 130 with a very low input impedance. In fact, it is preferred for the input impedance to be much lower than the optimum source impedance, e.g. a maximum ratio of optimum source impedance to input impedance. The reason for maximizing that ratio is so that the input impedance itself may be transformed, via a matching network, to present a high impedance to the coil 124, while at the same time, the relatively low source impedance 127 of the coil 124 is matched to the higher optimum source impedance of the preamplifier 130.

As an illustrative example, the preamplifier 130 includes an input stage 131 based on a Gallium-Arsenide (GaAs) field effect transistor (FET) 132. An input line 133 for the preamplifier 130 is coupled to the gate of the GaAs FET 132 via an adjustable L-C network 135. The resulting input stage 131 exhibits an optimum source impedance of approximately 50 ohms, while the L-C network 135 is adjusted to present an input impedance, at the frequency of interest, as low as practical. In this example, an input impedance of approximately 1.5 ohms is assumed. The output of GaAs FET 132 is then amplified in further stages 136 to produce the output signal 121.

Matching of the preamplifier 130 to the coil 124 is performed by a transmission line segment 137. The transmission line segment 137 has a length equal to one quarter wavelength ($\lambda/4$) at the frequency of interest. As is known in the art, a quarter-wave transmission line segment transforms the impedance seen at one end ($R_{end1}$), with respect to the impedance connected to the other end ($R_{end2}$), according to the formula:

$$R_{end1} = \frac{Z_o^2}{R_{end2}} \qquad (23)$$

or equivalently:

$$Z_o = \sqrt{R_{end1} * R_{end2}} \qquad (24)$$

Where: $Z_o$ is the characteristic impedance of a quarter-wave transmission line segment

A typical value for the source impedance 127 of the coil 124 is approximately 4 ohms. In order to match that 4 ohm source impedance to the optimum source impedance of 50 ohms for amplifier 130, Equation (24) yields a value for $Z_o$ of $\sqrt{200}$, or 14.14 ohms. Then, with a value of 14.14 ohms, the impedance seen across the gap 117 of coil 124 is obtained by applying Equation (23) to yield a value of 133 ohms. Therefore, the effective impedance connected across the coil 124 is 133 ohms, or approximately 2.6 times greater than the customary 50 ohms used with prior coil/preamplifier combinations. Similarly, that factor of a 2.6 increase impedance results in the desired effect of a corresponding decrease in the current circulating in coil 124.

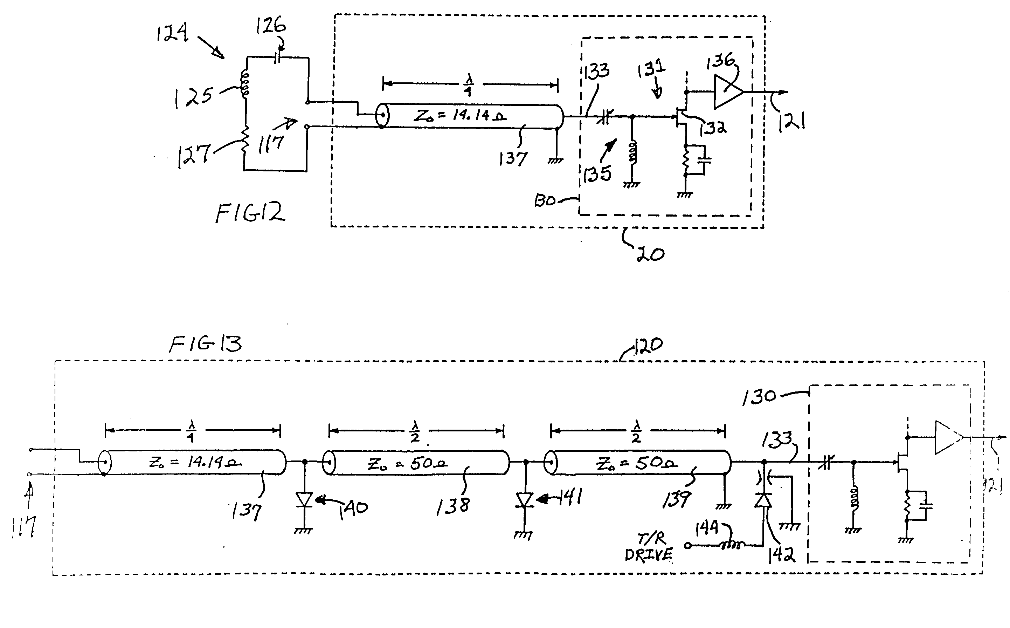

Referring to Figure 13, an alternative embodiment of the PREDAMP circuit 120 includes the same low impedance, low noise preamplifier 130 and the same quarter-wave transmission line segment 137 as in the previous embodiment. The embodiment of Figure 13 further includes half-wave transmission line segments 138 and 139, each with a characteristic impedance equal to the optimum source impedance of the preamplifier, or 50 ohms in this case. The half-wave transmission line segments 138 and 139 are therefore matched to the preamplifier 130 on one end and the quarter-wave transmission line segment 137 on the other end, and so do not effect the transformation of impedances, or PREDAMP effect, as described above.

One purpose of the half-wave transmission line segments 138 and 139 is to permit the insertion of clamping PIN diodes 140 and 141 to attenuate high voltages during the transmit phase. A third PIN diode 142 connects to preamplifier input 133 through an inductor 144. The inductor 144 is in turn driven by a transmit/receive (T/R) drive signal, which places a very low impedance clamp, on the order of .25 ohms, on the input 133 to preamplifier 130 during transmit. That lower impedance when clamped translates, according to Equations (23) and (24) above, into approximately 800 ohms across terminals 117 of the coil 124 (not shown in Figure 13), which is even more effective in reducing currents in the coil during transmit.

It should be apparent that even greater increases in effective coil impedance are possible by increasing the optimum-source-impedance-to-input-impedance ratio. By making that ratio higher, and appropriately adjusting $Z_o$, circulating currents in the coil 124 can be further reduced. An if that ratio is made high enough, coupling of noise currents to other coils is effectively eliminated by the PREDAMP circuit 120 alone, regardless of the relative positioning of the coils. This latter aspect is particularly useful in conjunction with flexible coil arrays in which the relative positioning of the coils are not known in advance and vary from patient to patient.

Referring again to Figure 11, the use of a PREDAMP circuit 120 connected to each of the coils 111a - 111d greatly reduces the coupling of noise between coils, as just described. It is therefore practical using this invention to simultaneously acquire high SNR images from each coil 111a - 111d independently. A further object of this invention, described below, is to combine the independent images so obtained into a composite image with both an enlarged field of view as compared to a single coil, and an enhanced SNR.

The independent images from the coils 111a - 111d are formed using conventional quadrature phase detectors and baseband processors (not shown) for each coil. The result is a set of complex numbers, describing each volume element (voxel) in the image, stored in a conventional processing apparatus (not shown), which are processed according to the below described method.

In particular, the method of this invention is most effective for arrays of coils in which the coils are not all in the same plane. In that case, as in the array of Figure 11, a signal produced in the common field of view of the array will be received with varying phase shifts in the different coils of the array. One objective of this invention is to combine those signals to achieve the optimum SNR at all locations in a multiple surface coil array by taking advantage of the spatially dependent phase shifts therein. Better than $\sqrt{2}$ noise improvement (ie., the quadrature reception advantage) are possible at certain locations near the coils.

In the complex image data obtained as described above, there are spatial variations of phase from pixel-to-pixel in a given individual image as well as phase variations from image-to-image for a fixed pixel. These phase variations effect how the correlated noise is incorporated in the composite image.

In general, when a signal 121 has a high SNR, the signal serves as a reference for the phase detection of the noise. That is, only the component of noise that is instantaneously in phase with the signal tends to alter the magnitude of the image. The component of noise that is out of phase with the signal tends to change the phase but not the magnitude of the image. A particular effect arises from the inhomogeneity of the B1 field generated by a unit current in each individual coil. The signal E induced in the coil by a given voxel is given by [25]

$$E= -\frac{d}{dt}\{\underline{B1}_{xy} \cdot \underline{m}\} \qquad (25)$$

where: $\underline{m}$ is the nuclear magnetic moment of the voxel, and
$\underline{B1}_{xy}$ is the xy vector component of B1.

When a body coil (not shown) is used to excite the nuclei, $\underline{m}$ initially points in the same direction for all voxels, i.e., they have a common phase reference such as the y-axis in the laboratory frame. The scalar product introduces a phase factor due to the variations in direction of $B1_{xy}$ from voxel to voxel and from coil to coil. Thus for two adjacent surface coils, the signals for a given pixel may have very different phases in the two images. Hence, correlated noise induced in both coils need not be detected with the same phase reference for a given pixel in both images. The treatment of the correlated noise can differ from pixel to pixel in the composite image. In some cases, the correlation can be nulled or reversed in certain pixels. For example, at a pixel where the B1 of one coil is anti-parallel with the B1 of the other coil, the signals are 180 degrees out of phase; so the correlated noise will add to the magnitude of one coil's image and subtract from the magnitude of the other coil's image. Combining the two images will eliminate the correlated noise in that pixel.

The method of the present invention combines the images acquired simultaneously from each coil 111a - 111d of the array by using both magnitude and phase information from each voxel. In the illustrative example that follows, the combination of two images from two of the coils 111a - 111d is explained in detail using subscripts i=1,2. Later, it is explained how the exemplary two coil case can be expanded to combine the images from an arbitrary number of coils.

Each coil has a signal $S_i$ and noise $n_i$ i assumed to be

$$s_i= S_i e^{j\theta}i \qquad n_i= N_i e^{j\phi}i \qquad (26)$$

$S_i(\underline{r})$ is proportional the magnitude of $B1_{xy}$ and therefore depends on the location r of the pixel. $\theta(\underline{r})$ equals the angle between $B1_{xy}$ and the initial direction of $\underline{m}$. The noise does not have any explicit spatial dependence because it results essentially from an integration over the sample volume. $N_i$ and $\phi_i$ are each random variables which vary from one data acquisition to the next. A typical probability distribution for N and $\phi$ may be, for example:

$$p(N,\phi)drd\phi = \frac{1}{\sigma^2} e^{-(N^2/\sigma^2)} 2NdN \left(\frac{d\phi}{2\pi}\right) \qquad (27)$$

This corresponds to Gaussian distributions for both the real and imaginary components of N and a uniform distribution of $\phi$ independent of N. For this case, $\langle N^2 \rangle = \sigma^2$, where the symbols $\langle \rangle$ denote an ensemble average over many data acquisitions.

Although both $n_1$ and $n_2$ are random variables, they may be correlated. The average noise power for the sum of two noise voltages is

$$\langle\text{noise power}\rangle = \langle(n_1+n_2)(n_1+n_2)^*\rangle = \langle n_1{}^2\rangle + \langle n_2{}^2\rangle + 2\langle n_1 n_2 \cos(\phi_1-\phi_2)\rangle \tag{28}$$

In Equation (6), the last term $2\langle n_1 n_2 \cos(\phi_1-\phi_2)\rangle$ accounts for any correlation between the two noises.

There are several methods according to this invention to combine the complex image data pixel by pixel. The simplest method, referred to herein as the mean image, takes a weighted sum of the image magnitudes for each pixel. The composite signal with noise included is given by:

$$(\text{mean image}) = |s1+n1| + \eta|s2+n2| \tag{29}$$

where $\eta$ is the weighting factor.

The second approach, referred to herein as the rms image, takes the square root of a weighted sum of the two image magnitudes squared. That is:

$$(\text{rms image}) = \{(s_1+n_1)(s_1+n_1)^* + \eta(s_2+n_2)(s_2+n_2)^*\}^{1/2}. \tag{30}$$

where: * denotes the complex conjugate operation

To determine the effect of noise on the composite image, the variance of the magnitude of each pixel is calculated according to the formula:

$$\text{variance} = \langle\text{mag}^2\rangle - \langle\text{mag}\rangle^2 \tag{31}$$

with mag= (mean image) or mag= (rms image). The signal-to-noise ratio SNR is given by the noiseless signal divided by the square root of the variance. The SNR is then optimized by finding its maximum with respect to the weighting factor, $\eta$. The calculated SNR and optimum $\eta$, designated $\eta_{opt}$ can be derived from the above equations assuming a high SNR. Omitting those calculations for simplicity, the results are shown below for both the mean image and the rms image.

Using the mean image approach, the calculations give:

$$SNR = \frac{(S_1 + S_2\eta)\sqrt{2}}{\sqrt{\langle N_1{}^2\rangle + \langle N_2{}^2\rangle\eta^2 + 2\eta x}} \tag{32}$$

$$\eta_{opt} = \frac{S_2\langle N_1{}^2\rangle - S_1 x}{S_1\langle N_2{}^2\rangle - S_2 x} \tag{33}$$

where:

$$x = \cos(\theta_1-\theta_2)\langle N_1 N_2 \cos(\phi_1-\phi_2)\rangle + \sin(\theta_1-\theta_2)\langle N_1 N_2 \sin(\phi_1-\phi_2)\rangle$$

For the rms image approach, the results are:

$$SNR = \frac{(S_1^2 + S_2^2 \eta)\sqrt{2}}{\sqrt{S_1^2 \langle N_1^2 \rangle + S_1^2 \langle N_2^2 \rangle \eta^2 + 2S_1 S_2 \eta x}} \qquad (34)$$

$$\eta_{opt} = \frac{S_1(S_2 \langle N_1^2 \rangle - S_1 x)}{S_2(S_1 \langle N_2^2 \rangle - S_2 x)} \qquad (35)$$

Note that the $\eta_{opt}$ for the rms image is equal to a factor $(S_1/S_2)$ times the optimum $\eta$ for the mean image. Combining Equations (32) and (33) can be seen to yield the same SNR for the composite image as the combination of Equations (34) and (35).

The expression denoted by x includes the correlation of the noise in the two coils. The angle $\theta_1-\theta_2$ is equal to the angle between the $\underline{B1}_{xy}$ vectors of the coils at a given voxel. The term $\langle N_1 N_2 \cos(\phi_1-\phi_2)\rangle$ gives the traditional correlation term as in Equation (6). The term $\langle N_1 N_2 \sin(\phi_1-\phi_2)\rangle$ is assumed to be equal to zero. Based on that assumption, the correlated noise is controlled by the coil geometry through the $\cos(\theta_1-\theta_1)$ factor. At points where the flux lines from the two coils are orthogonal, $\cos(\theta_1-\theta_2)$ is zero and the two coils have the SNR improvements due to quadrature reception for the voxel. If the flux lines are anti-parallel, then $\cos(\theta_1-\theta_2) = -1$ and the SNR is even better than for quadrature detection (provided there is a positive noise correlation). When x=0, the optimum for the rms image equals one and the optimum image corresponds to taking the square root of the sum of the individual SNR's squared.

In order to construct the composite image with optimum SNR, the weighting factor $\eta$ must be evaluated for each pixel. Relative values for $\langle N_1 2\rangle$, $\langle N_2 2\rangle$, and $\langle N_1 N_2 \cos(\phi_1-\phi_2)\rangle$ can be obtained by taking several data acquisitions during a prescan period with zero transmitter power. The values of $S_1$, $\theta_1$, $S_2$, and $\theta_2$ can be taken from the magnitude and phase of the images reconstructed for each individual coil provided no unequal phase shifts are introduced by the receiver channels. This procedure avoids the need to calculate the values and directions of $\underline{B1}_{xy}$ for each pixel. The latter calculation is not trivial for a coil array whose position or shape can vary from one patient to the next. For regions of low signal-to-noise, $\eta=1$ is a good initial approximation when using the rms image approach.

A coil array in which all coils are in the same plane is equivalent to taking $\theta_1 = \theta_2$ for all pixels. For the special case of a spinal probe made up of a single row of coils along the z-axis, $\theta_1$ equals $\theta_2$, and an optimal image is obtained according to the above described method of this invention. But more particularly, for coil arrays that extend tangentially around the body or head, $-1 \leq (\cos(\theta_1-\theta_2) \leq +1$, and the SNR obtained by the method of this invention will always equal or exceed the SNR produced by other prior methods which do not take into account the spatially imposed phase shifts. The amount of improvement in SNR depends on the relative signal strength, the degree of noise correlation, and the phase factor $\cos(\theta_1-\theta_2)$.

For the case where:

$$\langle N_1^2 \rangle = \langle N_2^2 \rangle \qquad (36)$$

$$\rho = \frac{<N_1 N_2 \cos(\phi_1 - \phi_2)>}{<N_1^2>} \qquad (37)$$

$$\beta = \frac{S_2}{S_1} \qquad (38)$$

$$c = \cos(\theta_1 - \theta_2) \qquad (39)$$

combining equations (12) and (13) gives:

$$SNR_{opt} = \sqrt{\frac{2S_1^2}{<N_1^2>}} \sqrt{\frac{1 - 2\beta\rho c + \beta^2}{1 - \rho^2 c^2}} \qquad (40)$$

The first factor of Equation (40) is the SNR obtained when only a single coil is used. The second factor is the enhancement due to optimum addition of the second coil's signal. The table below gives the enhancement factor EF for two values of $\rho$ for a range of $\beta$ and c.

| $\beta$ | c | EF($\rho$=.4) | EF($\rho$=.2) |
|---|---|---|---|
| 1.00 | +1 | 1.195 | 1.291 |
| | 0 | 1.414 | 1.414 |
| | -1 | 1.825 | 1.581 |
| .75 | +1 | 1.070 | 1.147 |
| | 0 | 1.250 | 1.250 |
| | -1 | 1.604 | 1.393 |
| .50 | +1 | 1.006 | 1.046 |
| | 0 | 1.118 | 1.118 |
| | -1 | 1.401 | 1.229 |

When $\cos(\theta_1 - \theta_2) = 0$, the dependance on the correlation coefficient $\rho$ drops out and the enhancement starts at $\sqrt{2}$ for equal signals and decreases as the second coil's signal strength (or SNR) decreases. When $\cos(\theta_1 - \theta_2)$ is greater than zero, the multi-coil enhancement becomes less effective as the correlations increase. The converse is true for negative values of $\cos(\theta_1 - \theta_2)$; the enhancement factor can exceed the $\sqrt{2}$ value given by quadrature reception. Hence, the SNR can be substantially improved in certain regions of the image by incorporating the coil induced phase shifts in the composite image reconstruction. In the table above, up to 54% improvement in SNR occurs when the cosine factor is not ignored.

Considering the additional geometric dependence of each pixel's SNR via the $\cos(\theta_1-\theta_2)$ term, the best choice of coil size and spacing for a wrap around body or head multiple coil array may need to be determined empirically. By using a PREDAMP circuit according to the present invention, as described above, to prevent cross coupling of the coils, the need to overlap coils to reduce their mutual inductance is reduced. A calculated SNR averaged over the desired imaging volume could actually be optimized by varying the configuration of individual coils in the array. The SNR calculation would combine coil loading and noise correlations with the geometric factors arising from spatial dependance of each coil's B1 vector.

It should then be apparent to those skilled in the art that the method of this invention can then be extended to any number of coils by utilizing appropriate matrix operations instead of the specific two coil example given. For example, for N surface coils, let $\underline{S}$ be an N dimensional vector $(S_1, S_2, .... SN)$ corresponding to the signals for a given voxel from N surface coils. Let $\rho$ be an NxN matrix whose ij element is:

$$\rho ij = \langle N_i N_j \cos(\phi_i - \phi_j) \rangle \cos(\theta_i - \theta_j).$$

Then the composite signal should be:

$$\text{COMPOSITE SIGNAL} = \{S \cdot (\rho^{-1}) . S\}^{1/2}.$$

For uncorrelated noise, this reduces to the square root of the sum of the squares of the SNR for each coil.

While several presently preferred emodiments of our novel multiple surface coil arrays and methods for NMR imaging with such arrays and for reducing interaction between elements of such arrays, have been described in detail herein, many variations and modifications will now become apparent to those skilled in the art. It is our intent therefore, to be limited only by the scope of the appending claims and not by the specific details and instrumentalities presented by way of explanation of the embodiments described herein.

## Claims

1. A nuclear magnetic resonance (NMR) signal acquisition apparatus comprising:

   a first coil having a source impedance $R_S$;

   a first preamplifier having an optimum source impedance $R_{opt}$ and an input impedance $R_{in}$, with $R_{in}$ being less than $R_{opt}$; and

   first matching means for connecting the first coil to the first preamplifier such that the source impedance of the first coil $R_S$ is transformed by the matching means to be approximately equal to the optimum source impedance $R_{opt}$ at the point of connection between the first matching means and the first preamplifier, and the low input impedance of the first preamplifier is transformed by the first matching means to be a value higher than the optimum source impedance $R_{opt}$ at the point of connection between the first matching means and the first coil.

2. The NMR signal acquisition apparatus of claim 1 in which the first matching means comprises a transmission line segment having a length approximately equal to one quarter of a wavelength at the NMR signal frequency.

3. The NMR signal acquisition apparatus of claim 2 in which the quarter-wavelength transmission line segment has a characteristic impedance $Z_o$ determined by the equation:

$$Z_o = \sqrt{R_S R_{opt}}$$

4. The NMR signal acquisition apparatus of claim 2 in which the first preamplifier includes an input stage comprising an inductor-capacitor (L-C) network for establishing the input impedance of the preamplifier at the NMR signal frequency.

5. The NMR signal acquisition apparatus of claim 3 which further includes at least one half-wavelength transmission line segment interposed between the first matching means and the first preamplifier and clamping means attached to at least one end of the half-wavelength transmission line segment for clamping high voltages occurring during a transmit cycle of the NMR signal acquisition, wherein the half-wavelength transmission line segment has a characteristic impedance approximately equal to the optimum source impedance $R_{opt}$.

6. The NMR signal acquisition apparatus of claim 1 in which the ratio of the optimum source impedance $R_{opt}$ to the input impedance $R_{in}$ is greater than 20.

7. The NMR signal acquisition apparatus of claim 1 in which the apparatus includes an array of at least one additional coils, at least one additional matching means, and at least one additional preamplifiers, each additional coil, additional matching means, and additional preamplifier being similar to the first coil, first matching means, and first preamplifier, respectively, whereby mutual coupling of noise currents between the coils is reduced by the high impedance presented by each respective matching means to the associated coil.

FIG. 1

FIG. 1a

FIG. 1b

FIG. 1c

FIG. 2a

FIG. 2b

FIG. 3

EP 0 696 744 A2

FIG. 5

FIG. 4

FIG. 4a

FIG. 7

(a) 25 S S O Z

(b) 25a 25' 25b S S 1.9S Z

(c) 25a 25b 25c 25d S S S S 3.7S Z 25''

O I

(d) 30 S 3.7S Z O

FIG. 6

20-1 21-1 $\phi_1$ 1:$n_1$ 22-1 20-i 21-i $\phi_i$ 1:$n_i$ 22-i 20-n 21-n $\phi_n$ 1:$n_n$ 22-n

20a Vout 20b

20

FIG. 8

FIG. 9a

FIG. 9b

FIG. 10

FIG 11

EP 0 696 744 A2

FIG 12

FIG 13